# NEW EUROPEAN PATENT SPECIFICATION

(11) **EP 1 260 205 B2**
(45) Date of publication and mention of the opposition decision: **28.11.2012**
(45) Mention of the grant of the patent: 27.08.2008
(21) Application number: 02253583.5
(22) Date of filing: 22.05.2002
(51) Int. Cl.: A61F 13/472, A61F 13/47, A61F 13/474

(54) **Adaptable absorbent articles**
Anpassbare absorbierende Artikel
Articles absorbants adaptables

(30) Priority: 23.05.2001 US 863529; 25.09.2001 US 962425
(43) Date of publication of application: 27.11.2002
(60) Divisional application: 06076526.0
(73) Proprietor: McNeil-PPC, Inc., Skillman, New Jersey 08558 (US)
(72) Inventor: Fernandez-Kleinlein, Elena, 40597 Düsseldorf (DE); Mangin, Sophie, 2284 AT Rijswijk (NL)
(74) Representative: Mercer, Christopher Paul

(56) References cited:
- EP-A- 1 008 333
- EP-A- 1 336 397
- WO-A-00/13633
- WO-A-95/15139
- WO-A-96/02217
- DE-A- 19 834 785
- DE-U- 29 614 542
- DE-U1- 29 614 542
- GB-A- 2 100 609
- US-A- 5 683 373
- US-A- 5 704 929
- US-A- 5 704 929
- US-A- 5 713 886
- US-A- 5 728 085
- US-A- 5 729 835

## Description

### Background of the Invention

This invention relates to absorbent articles, such as pantiliners, sanitary napkins, and incontinence pads. More particularly, the present invention relates to adaptable absorbent articles.

### Background of the Invention

Currently, when an absorbent article for sanitary protection, such as a pantiliner, a sanitary napkin, or an incontinence pad, is taken from its packaging, it is a single size that is used for a variety of individual body shapes and sizes, in addition to a variety of garment styles. A product that offers superior comfort, fit, and protection for one user or style of underwear may be deficient for another user or style of underwear.

For example, typical pantiliners are designed for use with garments having a full sized crotch portion, e.g., briefs and bikinis. However, such pantiliners do not readily lend themselves for use with garments having an abbreviated crotch portion, e.g., thong garments. As a result, many users purchase multiple types of sanitary protection depending on the underwear styles they wear.

U.S. Patent No. 5,704,929 (Bien) discloses an absorbent article having removable portions that can reduce the dimensions of the article. The disclosed preferred embodiment is a pantiliner that can be adjusted in size by tearing the absorbent article along one or more perforation lines and removing the portions that lie outboard the perforation lines. The resultant pantiliner, however, is designed for garments having a full sized crotch portion and is not adaptable for thong garments.

U.S. Patents Nos. 4,596,570 (Jackson et al.) and 4,597,759 (Johnson) disclose sanitary napkins capable of being elongated. Jackson et al. unfolds pleats at the longitudinal ends and Johnson adds a second absorbent element to a first element.

U.S. Design Patent No. D368,519 (Harrison et al.) discloses a pantiliner having a perforated section in the posterior portion. The embodiments shown have posterior portions that are narrower than the anterior portions.

DE 198 34 785 discloses a sanitary napkin manufactured such that it is fordable into a substantially tapered shape to fit to the individual form of the wearer.

As evident from the above, users that wear various types of garments often have the expense and bother of purchasing assorted sized products to meet their needs. Often, a user compromises and chooses only one size of sanitary protection even though that selection may be less than ideal.

What is needed, therefore, is an absorbent article that offers sanitary protection while also being adaptable to fit various garments.

### Summary of the Invention

This invention relates to a user adaptable absorbent article having a silhouette with a first end and a second end, wherein the second end being in opposite relation to the first end, and a first longitudinally extending edge opposed to a second longitudinally extending edge, the longitudinally extending edges connecting the first end and the second end; a layered portion having an absorbent core and a backsheet; and means for folding a portion of the absorbent article located either from or between at least one of the longitudinally extending edges and at least one of the ends, wherein the fold avoids the absorbent core.

### Brief Description of the Drawings

Figure 1 is a plan view an absorbent article according to the present invention depicted in the crotch portion of a conventional garment;
Figure 2 is a plan view of the absorbent article of Figure 1 depicted in the crotch portion of a thong garment, and
Figure 3 is a plan view of an additional embodiment of an absorbent article according to the present invention depicted in the crotch portion of a conventional garment.
Figure 4 is a plan view of an absorbent core of the present invention.
Figure 5 is a plan view of an absorbent core of the present invention.

### Detailed Description of the Invention

The present invention is directed to an absorbent article having a silhouette with a first end and second end, wherein the second end is in opposite relation to the first end. A pair of opposed longitudinally extending edges connect the first end to the second end. The present invention also includes a layered portion having a backsheet and an absorbent core. The shape of the absorbent core is such that a fold line from at least one longitudinally extending edge to the second end permits a portion of absorbent article to fold without the absorbent core being present in the folded section. In an alternate embodiment of the present invention, the fold line extends between at least one longitudinally extending edge and the second end.

As used herein, the term thong garment includes, but is not limited to, thong underwear, thong swimming suit bottom, G-strings, Rio cut underwear, Rio-cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself.

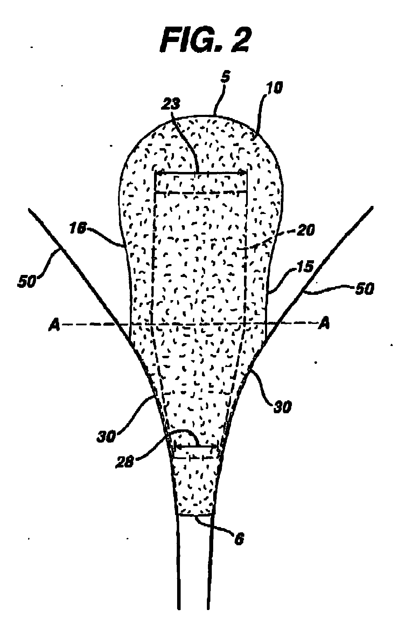

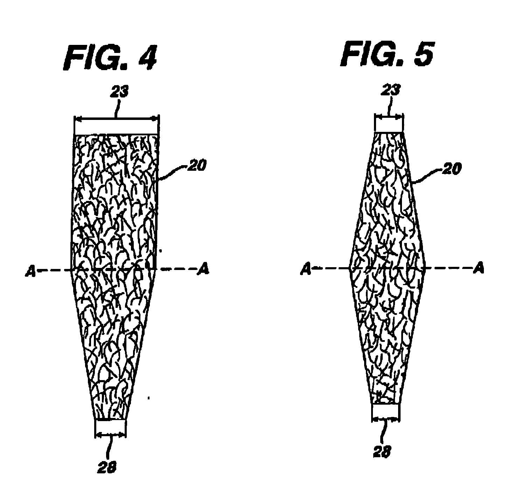

As used herein, the term asymmetrical silhouette means that the silhouette is asymmetrical about the A-axis, as depicted in Figs. 2 and 4.

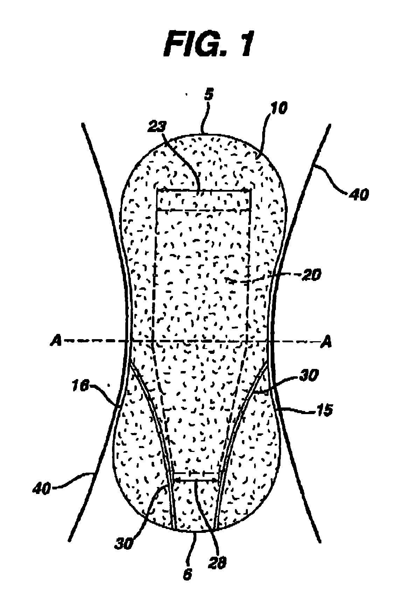

As used herein, the term symmetrical silhouette means that the silhouette is symmetrical about the A-axis, as depicted in Figs. 1, 3 and 5.

The absorbent core or layer of the present invention may contain any known absorbent materials including, but not limited to, absorbent fibers, such as cellulose fibers, e.g., wood pulp, regenerated cellulose fibers, and cotton fibers, rayon fibers, superabsorbent polymers, e.g., fibers or particles, other naturally occurring absorbent materials, e.g., peat moss, and other synthetic absorbent materials, such as foams and the like. The absorbent layer may also include one or more of the following: thermoplastic binder fibers, latex binder, perfumes, or odor-controlling compounds. The absorbent layer may be compressed or uncompressed, embossed, or calendered.

The backsheet of the present invention is a body fluid impervious material, typically referred to as a "barrier," at least substantially impermeable to liquids, and its exterior forms the garment-facing surface of the absorbent article. The backsheet may be any thin, flexible, body fluid impermeable material, such as a polymeric film, e.g., polyethylene, polypropylene, or cellophane, or a normally fluid pervious material that has been treated to be impervious, such as impregnated fluid repellent paper or non-woven material, including non-woven fabric material, or a flexible foam, such as polyurethane or cross-linked polyethylene. The thickness of the backsheet when formed from a polymeric film typically is 0.001 to 0.002 inch (0.00254 to 0.00508 cm).

Optionally, the backsheet may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

In Figures 1-3 fold line 30 is shown. Fold line 30 may be formed stitching, embossing, crimping, and perforation. It is within the scope of the present invention to include one or more fold line 30. For example, the absorbent article may have 2, 3, 4 or more fold lines.

As used herein, the term perforation is intended to mean a line of cuts, scores, embossing, crimping and the like. The choice of perforating methods is dependent on the materials and amount of cut desired. Commonly used methods for perforation include knife cutting, ultrasonic cutting, embossing, and sealing. A partially cutting knife will produce clean cuts through materials with parts of the perforation line not cut. For a sealing or embossing tool, the material would be crushed or fractured along the perforation line to form a stress concentrated area. Such a stress concentrated area may optionally be used to remove a portion of the absorbent article.

Optionally, a perforation may be completely through the layered portion of the absorbent article or almost all the way through, whichever makes is desired. Perforation may optionally be done when the absorbent article is complete, with or without the paper release strip. It is not necessary to perforate the release paper, if present although the release paper may also be perforated. The perforations, however, must not compromise the strength of the fold line, i.e, permit unwanted tearing, when the absorbent article is used with a thong garment.

The overall dimensions of the absorbent article of the present invention are preferably as follows. The length is preferably in the range of 5 to 8 inches (12 to 20 cm). The maximum width of the anterior portion is preferably in the range of 2 to 3 inches (5 to 8 cm). The thickness of the absorbent article is preferably in the range of 0.04 to 0.2 inches (0.1 to 0.5 cm).

Although not required, the absorbent article of the present invention may include a cover overlaying the absorbent core. The exterior of the cover would then form the body-facing side of the absorbent article. The cover may be formed from any fluid pervious material that is comfortable against the skin and permits fluid to penetrate to the absorbent core, which retains the fluid. The cover should retain little or no fluid to provide a relatively dry surface next the skin when in use. A variety of cover materials are known in the art, and any of these may be used. For instance, the cover may be a fibrous non-woven fabric made of fibers or filaments of polymers, such as polyethylene, polypropylene, polyester, or cellulose, and combinations thereof. Alternatively, the cover may be formed from an apertured polymeric film. The thickness of the cover may vary from 0.001 to 0.062 inch (0.0025 to 0.016 cm), depending on the material chosen.

Generally, the optional cover is a single sheet of material having a width sufficient to form the body-facing surface of the absorbent article. Preferably, the cover is longer and wider than the absorbent core.

Optionally, the absorbent article of the present invention may include a transfer layer. If included in the absorbent article, the transfer layer may be made of any known material that will take up fluid and then distribute and release it to an adjacent absorbent core or layer for storage. Preferred transfer layers have a relatively open structure that allows for movement of fluid within the layer. Suitable materials for such transfer layers include fibrous webs, resilient foams, and the like.

The transfer layer is able to accept fluid and allow passage of the fluid through its mass to be absorbed by an adjacent absorbent core. The mass of materials making up the transfer layer may be absorbent, although the materials themselves may not absorb. Thus, transfer layers that are made of hydrophobic, nonabsorbent fibers may be able to accept large volumes of fluid into interfiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the interfiber void spaces in the fibrous transfer layer or the open cells in the foam transfer layer, function much like a container to hold fluid.

Preferred transfer layer fibrous webs are made of resilient, nonabsorbent materials to provide void volume and to allow for free movement of fluid through the structure. Transfer layers that are made from webs of mostly absorbent fibers absorb the fluid as it enters the structure and do not distribute it throughout the rest of the structure as efficiently as webs containing non-absorbent materials.

As is customary in the art, a paper release strip, which has been coated on one side, may be applied to protect adhesive that may be applied to the garment-facing side of the backsheet. The coating on the paper release strip, which may be silicone, reduces the adherency to the adhesive of the coated side of the release strip. The release strip can be formed from any suitable sheet-like material which, when coated, adheres with sufficient tenacity to the adhesive to remain in place prior to use but which can be readily removed when the absorbent article is to be used.

The adhesive applied to the garment facing side of the absorbent article may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include rapid setting thermoplastic "hot melt," rubber adhesives, two-sided adhesive tape, and the like.

Any or all of the cover, absorbent core, transfer layer, backsheet, and adhesive may be colored or transparent. Such coloring includes, but is not limited to, white, black, yellow, blue, orange, green, violet, and the like. Color may be imparted according the present invention though dying and/or pigmentation. Colorants used according the present invention include dyes and inorganic and organic pigments. The dyes include, but are not limited to, Azo dyes (e.g., Solvent Yellow 14, Disperse Yellow 23, Metanil Yellow), anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like.

Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

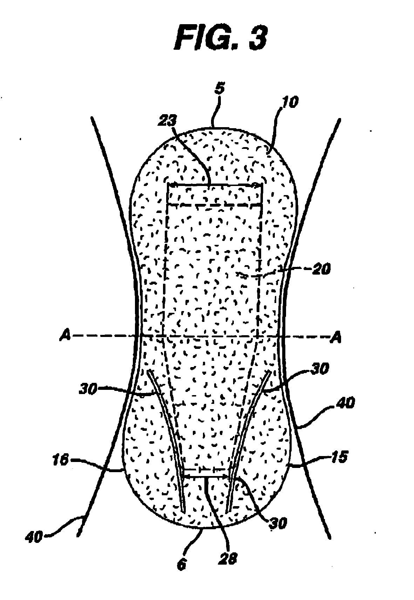

Turning to the Figures, Figures 1 and 3 depict an absorbent article 10 in a conventional garment 40 according to the present invention. The overall silhouette of the absorbent article is such that in it will fit comfortably within the confines of a conventional garment when not folded. Referring to Figure 1, absorbent article 10 includes asymmetric absorbent core 20 and fold line 30. Absorbent core 20 has first end width 23 and second end width 28.

Fold line 30 may extend from longitudinally extending edge 16, longitudinally extending edge 15, or both to the second end 6, as depicted in Figure 1 , or optionally first end 5. Optionally, fold line 30 may extend between longitudinally extending edge 16, longitudinally extending edge 15, or both and the second end 6, as depicted in Figure 3.

Turning to Figure 2, absorbent article 10 may be applied to the crotch of thong garment 50 by placing the garment-facing surface of the absorbent article 10 against the inside surface of the crotch of the thong garment. Absorbent article 10 may be folded along fold line 30. Depending on how the user folds absorbent article 10, a portion of the absorbent article between fold line 30 and longitudinally extending edges 15, 16, or both may be folded around the garment. The tapered longitudinal edge of absorbent core 20 permits folding without any perforation being present. While the taper depicted in Figure 2 is straight, any shape or length of taper can be used for absorbent core 20.

The absorbent article may include other known materials, layers, and additives, such as, foam layers, net-like layers, perfumes, medicaments, moisturizers, odor control agents, and the like, many examples of which are known in the art. The absorbent article can optionally be embossed with decorative designs using conventional techniques.

Figure 4 depicts absorbent core 20 being asymmetric about the A-axis. Figure 5 depicts absorbent core 20 being symmetric about the A-axis. For example, where absorbent core 20 is asymmetric, first end width 23 is greater than second end width 28, i.e., the ratio of first portion maximum width 23 to second end width 28 is greater than 1:1. Also, where absorbent core 20 is symmetric, first end width 23 is equal to second end width 28, i.e., the ratio of first portion maximum width 23 to second end width 28 is about 1:1. First portion edge width 23 can be from 5 mm to 45 mm. Second portion edge width 28 can be from 5 mm to 45 mm.

The precise shapes of the first portion and the second portion of the absorbent core may vary as desired. For example, the first portion may have the overall shape of a bulb, triangular, or round. Alternatively, the asymmetric absorbent core may include a midsection that may be tapered and narrow at a substantially continuous rate along its length or it may have a biconcave in shape. The midsection may also be of a narrow, uniform width like a stem.

Also contemplated herein include symmetrical absorbent articles having parallel longitudinal edges, dog bone- or peanut-shaped, and the like, including conventional pantiliners, sanitary napkins, incontinence devices.

## Claims

1. An absorbent article (10) which is a sanitary napkin, pantiliner or incontinence pad, said article defining an hourglass silhouette for use with conventional underwear in a first configuration and said article tapering toward at least one end of the article in a second configuration for use with thong underwear, said article comprising:
a first end (5) and a second end (6);
a first longitudinally extending edge (15) and a second longitudinally (16) extending edge; and
a backsheet;
**characterised in that** said absorbent article additionally comprises a core (20) having a first longitudinally extending edge and a second longitudinally extending edge, said first and second longitudinally extending edges of said core being spaced inwardly relative to the first and second longitudinally extending edges of said absorbent article; and wherein said absorbent article is foldable into said second configuration along a fold line (30) thus producing folds between at least one of the longitudinally extending edges and at least one of the ends with said folds avoiding the core.

2. The absorbent article according to claim 1, wherein said core tapers toward one end of the core.

3. The absorbent article according to claim 1, wherein said core tapers toward both ends of the core.

4. The absorbent article according to claim 1, wherein said core has a symmetric shape.

5. The absorbent article according to claim 1, wherein said core has an asymmetric shape.

6. The absorbent article according to claim 1 or 2, having a cover which is longer and wider than said core.

7. The absorbent article according to claim 6, wherein:
said first longitudinally extending edge of said core is spaced inwardly along its entire length relative to said first longitudinally extending edge of said cover, and
said second longitudinally extending edge of said core is spaced inwardly along its entire length relative to said second longitudinally extending edge of said cover.

8. The absorbent article according to claim 7, wherein said fold line is spaced inwardly relative to said first and second longitudinally extending edges of said cover in said first unfolded configuration.

## Patentansprüche

1. Absorbierender Artikel (10), welcher eine Damenbinde, eine Slipeinlage oder eine Inkontinenzeinlage ist, wobei der Artikel eine Sanduhrsilhouette zur Verwendung mit Unterwäsche in einer ersten Konfiguration beschreibt; und sich der Artikel wenigstens zu einem Ende des Artikels hin in einer zweiten Konfiguration zur Verwendung mit Tangaslips verjüngt, wobei der Artikel umfasst:
ein erstes Ende (5) und ein zweites Ende (6);
eine erste sich longitudinal erstreckende Kante (15) und eine zweite sich longitudinal erstreckende Kante(16); und
eine Rückschicht;
**dadurch gekennzeichnet, dass** der absorbierende Artikel zusätzlich einen Kern (20) umfasst, der eine erste sich longitudinal erstreckende Kante und eine zweite sich longitudinal erstreckende Kante umfasst, wobei die ersten und zweiten sich longitudinal erstreckenden Kanten des Kerns relativ zu den ersten und zweiten sich longitudinal erstreckenden Kanten des absorbierenden Artikels nach innen voneinander beabstandet sind; und wobei der absorbierende Artikel in besagte zweite Konfiguration faltbar ist entlang einer Faltlinie (30), so dass Faltungen zwischen wenigstens einer der sich longitudinal erstreckenden Kanten und wenigstens einem der Enden erzeugt werden, wobei die Faltungen den Kern vermeiden.

2. Absorbierender Artikel nach Anspruch 1, **dadurch gekennzeichnet, dass** sich besagter Kern zu einem Ende des Kerns hin verjüngt.

3. Absorbierender Artikel nach Anspruch 1, **dadurch gekennzeichnet, dass** besagter Kern sich zu beiden Enden des Kerns hin verjüngt.

4. Absorbierender Artikel nach Anspruch 1, **dadurch gekennzeichnet, dass** besagter Kern eine symmetrische Form aufweist.

5. Absorbierender Artikel nach Anspruch 1, **dadurch gekennzeichnet, dass** besagter Kern eine asymmetrische Form aufweist.

6. Absorbierender Artikel nach Anspruch 1 oder 2, eine Abdeckung umfassend, die länger und breiter als besagter Kern ist.

7. Absorbierender Artikel nach Anspruch 6, **dadurch gekennzeichnet, dass**
die erste sich longitudinal erstreckende Kante des Kerns nach innen über ihre gesamte Länge relativ zu besagter erster sich longitudinal erstreckenden Kante der Abdeckung beabstandet ist, und
besagte zweite sich longitudinal erstreckende Kante des Kerns nach innen über ihre gesamte Länge relativ zu besagter zweiter sich longitudinal erstreckenden Kante der Abdeckung beabstandet ist.

8. Absorbierender Artikel nach Anspruch 7, **dadurch gekennzeichnet, dass** besagte Faltlinie nach innen relativ zu besagter erster und zweiter sich longitudinal erstreckenden Kante der Abdeckung in besagter erster ungefalteter Konfiguration beabstandet ist.

## Revendications

1. Article absorbant (10) qui est une serviette hygiénique, un protège-slip ou une serviette contre l'incontinence, ledit article définissant une silhouette de sablier destinée à être utilisée avec un sous-vêtement classique dans une première configuration et ledit article se rétrécissant progressivement vers au moins une extrémité de l'article dans une seconde configuration destinée à être utilisée avec un string, ledit article comprenant :
- une première extrémité (5) et une seconde extrémité (6),
- un premier bord (15) s'étendant longitudinalement et un second bord (16) s'étendant longitudinalement ; et
- une feuille de dessous ;
**caractérisé en ce que** ledit article absorbant comprend de plus une partie centrale (20) ayant un premier bord s'étendant longitudinalement et un second bord s'étendant longitudinalement, lesdits premier et second bords s'étendant longitudinalement de ladite partie centrale étant espacés vers l'intérieur par rapport aux premier et second bords s'étendant longitudinalement dudit article absorbant ; et dans lequel ledit article absorbant peut se plier dans ladite seconde configuration le long d'une ligne de pliage (30) pour produire ainsi des plis entre au moins l'un des bords s'étendant longitudinalement et au moins l'une des extrémités avec lesdits plis évitant la partie centrale.

2. Article absorbant selon la revendication 1, dans lequel ladite partie centrale se rétrécit progressivement vers une extrémité de la partie centrale.

3. Article absorbant selon la revendication 1, dans lequel ladite partie centrale se rétrécit progressivement vers les deux extrémités de la partie centrale.

4. Article absorbant selon la revendication 1, dans lequel ladite partie centrale a une forme symétrique.

5. Article absorbant selon la revendication 1, dans lequel ladite partie centrale a une forme asymétrique.

6. Article absorbant selon la revendication 1 ou 2, ayant un recouvrement qui est plus long et plus large que ladite partie centrale.

7. Article absorbant selon la revendication 6, dans lequel :
- ledit premier bord s'étendant longitudinalement de ladite partie centrale est espacé vers l'intérieur le long de toute sa longueur par rapport audit premier bord s'étendant longitudinalement dudit recouvrement, et
- ledit second bord s'étendant longitudinalement de ladite partie centrale est espacé vers l'intérieur le long de toute sa longueur par rapport audit second bord s'étendant longitudinalement dudit recouvrement.

8. Article absorbant selon la revendication 7, dans lequel ladite ligne de pliage est espacée vers l'intérieur par rapport auxdits premier et second bords s'étendant longitudinalement dudit recouvrement dans ladite première configuration dépliée.
